# EUROPÄISCHE PATENTANMELDUNG

(11) **EP 2 002 807 A2**
(43) Veröffentlichungstag der Anmeldung: **17.12.2008**
(21) Anmeldenummer: 08010242.9
(22) Anmeldetag: 05.06.2008
(51) Int. Cl.: A61F 2/78

(54) **An- und Ausziehhilfe für Silikonliner**

(30) Priorität: 14.06.2007 DE 202007008526 U
(71) Anmelder: Münch, Thomas, 47167 Duisburg (DE)
(72) Erfinder: Münch, Thomas, 47167 Duisburg (DE)
(74) Vertreter: Röther, Peter

(57) **Zusammenfassung**

Die Anmeldung betrifft eine An- und Ausziehhilfe (1) für Silikonliner (9) von Arm- und/oder Beinprothesenträgern, die durch einen einseitig offenen Zylinder (2) gekennzeichnet ist, wobei diese Öffnung durch federnd gelagerte, nach außen verschwenkbare, über den Zylinderumfang gleichmäßig verteilte Laschen (3) gebildet ist, über deren Oberkanten ein in den Zylinder (2) eintauchender Sack (8) aus einem glatten Stoff gelegt ist.

## Beschreibung

Die Erfindung betrifft eine An- und Ausziehhilfe für Silikonliner von Arm- und/oder Beinprothesenträgern.

Derartige Silikonliner sind dem Bein- oder Armstumpf angepasste flexible Silikonhüllen, die ähnlich einem Strumpf den Stumpf fest umschließen. Am stumpfendseitigen Ende des Silikonliners ist ein Bolzen angeordnet, der in eine entsprechende Aufnahme in der anzuschließenden Prothese eingeführt wird und dort verriegelt wird. Somit ist die eigentliche Prothese sicher mit dem Silikonliner verbunden, wobei der Silikonliner wiederum durch Adhäsion die Verbindung zwischen Prothese und Stumpf ist.

Da der Silikonliner sehr straff sitzt, ist das An- und Ausziehen desselben sehr mühsam. Der Prothesenträger muss den oberen Rand des Silikonliners nach außen umschlagen und am Stumpf herabstreifen, bis der Stumpf freigegeben ist. Beim Anziehen muss ebenfalls erst der Rand des Silikonliners nach unten gerollt werden, damit der Prothesenträger den Stumpf einlegen kann, bevor der Rand wieder hoch geschoben werden kann. Dieser Vorgang ist natürlich insbesondere für Armamputierte mit erheblichen Schwierigkeiten verbunden.

Der Erfindung liegt daher die Aufgabe zugrunde, eine An- und Ausziehhilfe für Silikonliner anzugeben, bei der der Auf- und Abrollvorgang der Silikonlinerwand reibungsarm geschieht.

Die Erfindung löst diese Aufgabe gemäß dem kennzeichnenden Teil des Anspruchs 1 durch einen einseitig offenen Zylinder, wobei diese Öffnung durch federnd gelagerte, nach außen verschwenkbare, über den Zylinderumfang gleichmäßig verteilte Laschen gebildet ist, über deren Oberkanten ein in den Zylinder eintauchender Sack aus einem glatten Stoff gelegt ist.

Zum Anlegen des Silikonliners wird dieser nun in den Zylinder gesteckt und mitsamt dem Sack nach unten geschoben. Dabei ist der obere Rand des Sackes um die Oberkante des Zylinders nach unten herum gelegt. Auch der obere Rand des Silikonliners wird nun über die Oberkante des Zylinders umgeschlagen. Wird nun dieser umgeschlagene Rand nach unten geschoben, gleitet er auf dem glatten Stoff des Sackes fast reibungslos, bis das untere Ende des Silikonliners oben in der Zylinderöffnung erscheint. Die Person setzt nun den Stumpf auf den Innenboden des Liners und taucht mit dem Stumpf in den Zylinder ein, wobei sich der Liner wieder in die ursprüngliche gestreckte Form bewegt und sich um den Stumpf legt.

Der Ausziehvorgang verläuft analog.

Bei einer bevorzugten Ausführungsform gemäß Anspruch 2 ist vorgesehen, dass der aus dem Zylinder nach außen ragende obere Rand des Sackes um die Oberkante der Laschen herum nach unten geschlagen und durch Längsschlitze im Zylinder mit dem im Zylinder befindlichen Teil des Sackes verbunden ist. Somit wirkt der Sack beim Herunter- und Hochfahren im Zylinder wie ein Endlosband. Hierdurch wird dem Benutzer das Umschlagen des Sackrandes über den Zylinderrand erspart und die Gefahr vermieden, dass beim Herunterfahren der Sack ganz in den Zylinder eintaucht.

Um zu gewährleisten, dass der am unteren Ende des Silikonliners angeordnete Bolzen sich immer in der Zylinderlängsachse befindet, ist gemäß Anspruch 3 vorgesehen, dass in der Zylinderlängsachse eine Teleskopfeder angeordnet ist, auf deren Spitze innerhalb des Sackes ein Körper befestigt ist, der eine in der Zylinderlängsachse liegende, nach oben offene zylindrische Bohrung aufweist.

Beim Aufsetzen des Silikonliners auf den nach oben gezogenen Sackboden taucht der Bolzen in die zylindrische Bohrung des Körpers ein und wird gegen die Kraft der Feder beim Eintauchen des Stumpfs in den Silikonliner bzw. in den Zylinder exakt in der Zylinderlängsachse gehalten.

Die Teleskopfeder dient beim Ausziehen des Silikonliners zudem als zusätzliche Unterstützung.

Die Erfindung wird im folgenden anhand einer Zeichnung dargestellt und erläutert.

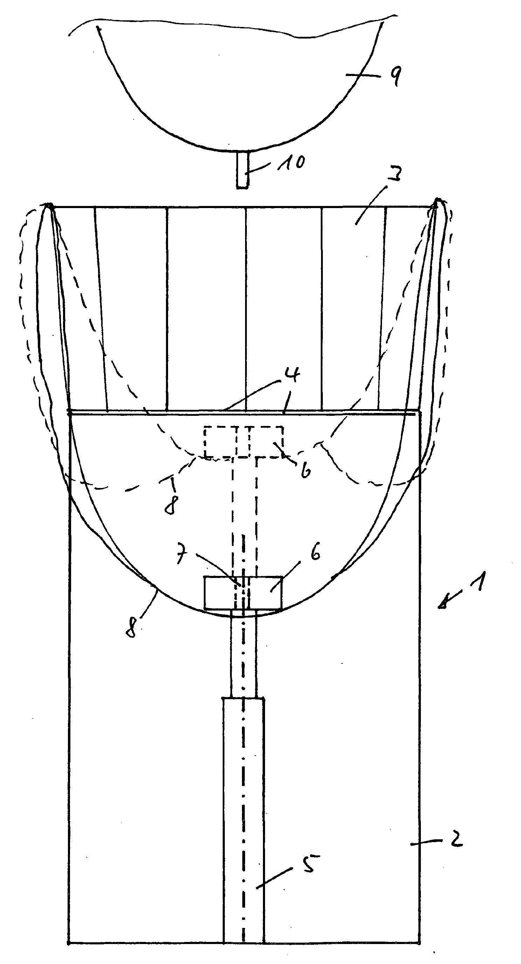

In der einzigen Figur ist im Querschnitt eine An- und Ausziehhilfe für Silikonliner von Arm- und/oder Beinprothesenträgern dargestellt und allgemein mit dem Bezugszeichen 1 versehen. Sie besteht aus einem oben offenen Zylinder 2, deren oberer Rand durch lamellenartige Laschen 3 gebildet ist, die nach außen verschwenkbar gegen Federkraft beispielsweise mittels Scharnieren 4 gelagert sind. Im Innern des Zylinders ist in der Zylinderlängsachse eine Teleskopfeder 5 angeordnet, auf deren Spitze ein Körper 6 mit einer zylindrischen Längsbohrung 7 angeordnet ist. Die Bohrung 7 liegt ebenfalls in der Zylinderlängsachse. Im innern des Zylinders 2 befindet sich ein Sack 8 aus einem glatten Stoff mit möglichst geringem Reibungswiderstand, beispielsweise Ballonseide. Der Sack 8 ist mit seinem oberen Rand über die Oberkanten der Laschen 3 herum nach unten umgeschlagen und über in der Zylinderwand vorgesehene Längsschlitze mit dem unteren Teil des Sacks 8, welcher sich im Zylinder 2 befindet, verbunden. Der Körper 6 sitzt hierbei auf der Innenseite des Sackbodens auf.

In der gestrichelten Darstellung des Sacks 8 befindet sich der Sackboden in der Nähe der Öffnung des Zylinders 2. Ein Silikonliner 9, an dem am unteren Rand ein Bolzen 10 zur Verbindung mit einer Prothese befestigt ist, wird nun auf den Sackboden aufgesetzt, derart, dass der Bolzen 10 in die Bohrung 7 eintaucht. Gegen die Kraft der Teleskopfeder 5 wird nun der Silikonliner 9 in den Zylinder nach unten gedrückt. Danach wird der obere Rand des Silikonliners um die Oberkante des Zylinders herumgeschlagen. Durch weiteres Herunterschieben des Silikonlinerrandes, der jetzt auf dem glatten Stoff des Sackes 8 gleitet, wird das Ende des Silikonliners wieder in Richtung Zylinderöffnung gefahren, so dass nun der Prothesenträger seinen Stumpf auf den Silikonlinerboden aufsetzen kann. Durch Einfahren des Stumpfes in den Zylinder 2 legt sich der Silikonliner 9 vollflächig um den Stumpf. Durch den reibungsmindernden Stoff des Sackes 8 kann dann die Person den mit dem Silikonliner umhüllten Stumpf aus dem Zylinder 2 herausziehen. Dabei ist sichergestellt, dass der Bolzen 10 sich genau an dem Ort befindet, an dem er zur Verbindung mit der Prothese sein soll.

Der Ausziehvorgang für den Silikonliner erfolgt analog.

## Patentansprüche

1. An- und Ausziehhilfe für Silikonliner von Arm- und/oder Beinprothesenträgern,
**gekennzeichnet durch** einen einseitig offenen Zylinder (2), wobei diese Öffnung **durch** federnd gelagerte, nach außen verschwenkbare, über den Zylinderumfang gleichmäßig verteilte Laschen (3) gebildet ist, über deren Oberkanten ein in den Zylinder (2) eintauchender Sack (8) aus einem glatten Stoff gelegt ist.

2. An- und Ausziehhilfe nach Anspruch 1,
**dadurch gekennzeichnet,**
**dass** der aus dem Zylinder (2) nach außen ragende obere Rand des Sackes (8) um die Oberkanten der Laschen (3) herum nach unten geschlagen ist und durch Längsschlitze im Zylinder (2) mit dem im Zylinder (2) befindlichen Teil des Sackes (8) verbunden ist.

3. An- und Ausziehhilfe nach Anspruch 1 oder 2,
**dadurch gekennzeichnet,**
**dass** in der Zylinderlängsachse eine Teleskopfeder (5) angeordnet ist, auf deren Spitze innerhalb des Sackes (8) ein Körper (6) befestigt ist, der eine in der Zylinderlängsachse liegende, nach oben offene zylindrische Bohrung (7) aufweist.
